# EUROPEAN PATENT APPLICATION

(11) **EP 2 412 697 A1**
(43) Date of publication of application: **01.02.2012**
(21) Application number: 10756148.2
(22) Date of filing: 25.03.2010
(51) Int. Cl.: C07C 69/76, A61K 31/235, A61P 19/10, C07C 213/04, C07C 213/08, C07C 217/28, C07D 303/22

(54) **PROCESS FOR PRODUCING CARBOXYLIC ACID COMPOUND**

(30) Priority: 26.03.2009 JP 2009077601; 02.04.2009 US 211712
(71) Applicant: Japan Tobacco, Inc., Tokyo 105-8422 (JP)
(72) Inventor: MATSUMOTO, Katsuya, Takatsuki-shi Osaka 569-1125 (JP); YOKOTA, Katsuyuki, Takatsuki-shi Osaka 569-1125 (JP); SHINAGAWA, Yuko, Takatsuki-shi Osaka 569-1125 (JP); INOUE, Teruhiko, Takatsuki-shi Osaka 569-1125 (JP)
(74) Representative: Vossius & Partner
(86) International application number: PCT/JP2010/055160
(87) International publication number: WO 2010/110352

(57) **Abstract**

The present invention provides the production method of carboxylic acid compound, as shown in Scheme I.

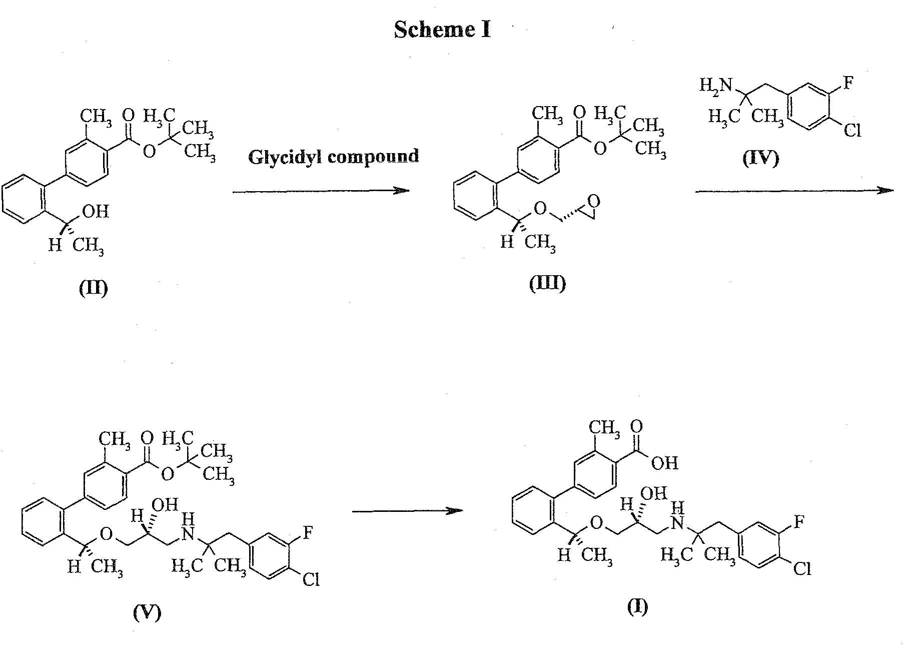

## Description

### Technical Field

The present invention relates to a production method of a carboxylic acid compound.

### Background Art

Patent document 1 describes a compound useful for the treatment of osteoporosis.

### [Citation List]

### [Patent Document]

Patent document 1: WO2004/094362

### Summary of the Invention

### Problems to be Solved by the Invention

An object of the present invention is to provide a novel production method of Compound I as represented by the structural formula

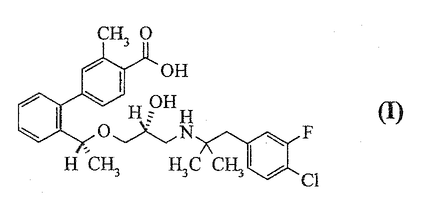

or a salt thereof, which is useful for the treatment of osteoporosis, and the like.

### Means of Soling the Problems

The present inventors have found a useful production method as represented by Scheme I

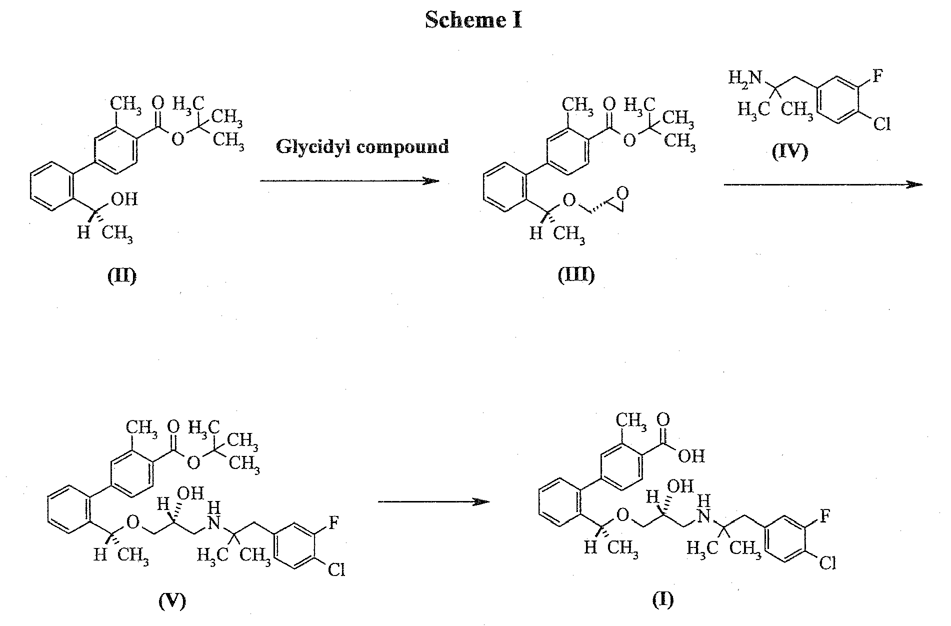

In Scheme I, each compound is as follows:
Compound I:
   2'-((1R)-1-{(2R)-3-[1-(4-Chloro-3-fluorophenyl)-2-methylpropan-2-ylamino]-2-hydroxypropoxy}ethyl)-3-methylbiphenyl-4-carboxylic acid
Compound II:
   tert-Butyl 3-methyl-2'-[(1R)-1-hydroxyethyl]biphenyl-4-carboxylate
Compound III:
   tert-Butyl 3-methyl-2'-[(1R)-1-((R)-oxiranylmethoxy)ethyl]biphenyl-4-carboxylate
Compound IV:
   [1-(4-Chloro-3-fluorophenyl)-2-methylprapan-2-yl]amine
Compound V:
   tert-Butyl 2'-((1R)-1-{(2R)-3-[1-(4-chloro-3-fluorophenyl)-2-methylpropan-2-ylamino]-2-hydroxypropoxy}ethyl)-3-methylbiphenyl-4-carboxylate
The present invention includes:

[1] A compound having the structural formula:

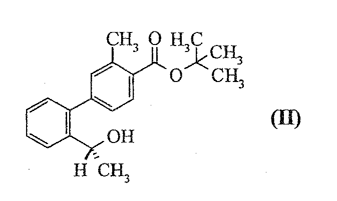

or a salt thereof.
[2] A compound having the structural formula:

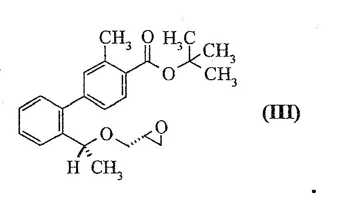

[3] A compound having the structural formula:

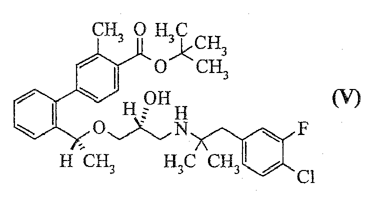

or a salt thereof.
[4] A method for the preparation of a compound having the structural formula:

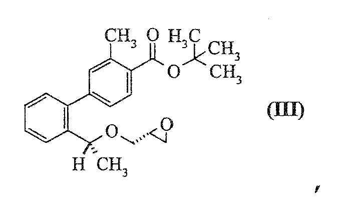

which comprises a step of subjecting a compound having the structural formula:

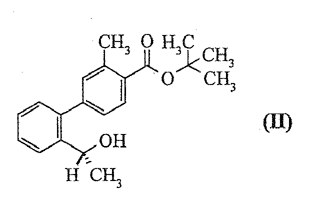

or a salt thereof to glycidylation.
[5] A method for the preparation of a compound having the structural formula:

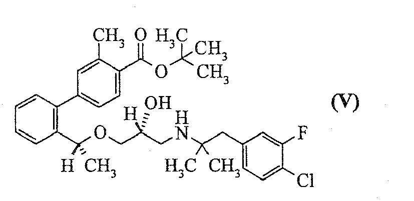

or a salt thereof, which comprises
(i) a step of subjecting a compound having the structural formula:

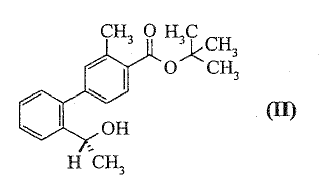

or a salt thereof to glycidylation to prepare a compound having the structural formula:

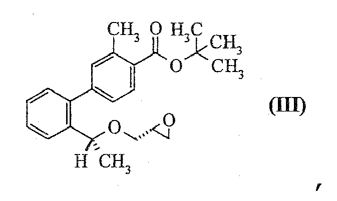

and
(ii) a step of reacting the formula III compound with a compound having the structural formula:

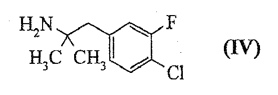

or a salt thereof.
[6] A method for the preparation of a compound having the structural formula:

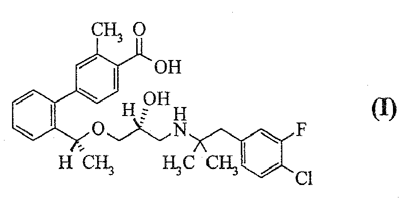

or a salt thereof, which comprises
(i) a step of subjecting a compound having the structural formula:

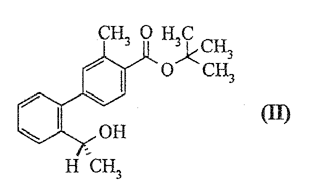

or a salt thereof to glycidylation to prepare a compound having the structural formula:

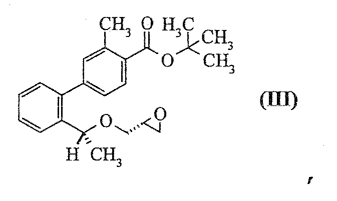

(ii) a step of reacting the formula III compound with a compound having the structural formula:

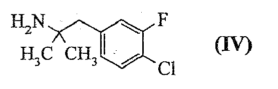

or a salt thereof to prepare a compound having the structural formula:

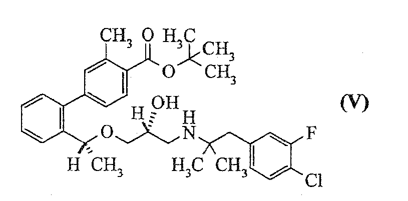

or a salt thereof, and
(iii) a step of subjecting the formula V compound or a salt thereof to alkali hydrolysis.
[7] A method for the preparation of a compound having the structural formula:

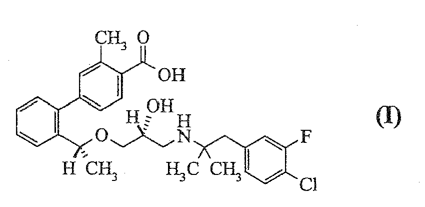

or a salt thereof, which comprises a step of subjecting a compound having the structural formula:

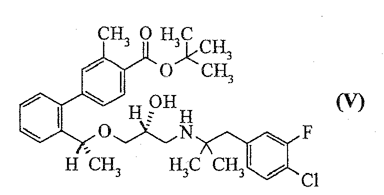

or a salt thereof to alkali hydrolysis.
[8] The method of the above-mentioned [6] or [7], wherein the step of subjecting a compound having the structural formula:

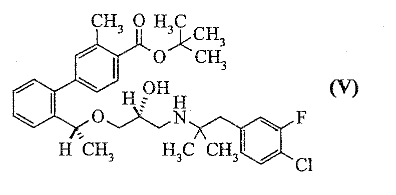

or a salt thereof to alkali hydrolysis is a step of subjecting a compound having the structural formula:

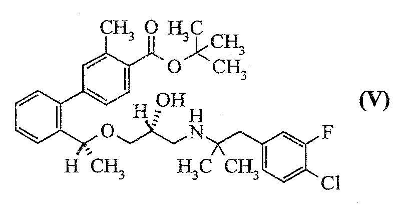

or a salt thereof to transesterification and then to alkali hydrolysis.

### Embodiments of the Invention

Certain compounds used in the present invention may be a salt of the compound.
In the present invention, the salt of the compound is preferably a pharmaceutically acceptable salt. Examples of the pharmaceutically acceptable salt include salts with inorganic acids, salts with organic acids, salts with inorganic bases, salts with organic bases, salts with amino acids, and the like.

Examples of the salt with inorganic acid include salts with hydrochloric acid, nitric acid, sulfuric acid, phosphoric acid, hydrobromic acid and the like.
Examples of the salt with organic acid include salts with oxalic acid, maleic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid, p-toluenesulfonic acid and the like.

Examples of the salt with inorganic base include sodium salt, potassium salt, calcium salt, magnesium salt, ammonium salt and the like.
Examples of the salt with organic base include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.
Examples of the salt with amino acid include salts with lysine, arginine, aspartic acid, glutamic acid and the like.

The invention also includes solvent addition forms ("solvates") of the compounds (e.g., compound I, compound II, compound III, compound IV or compound V) or a salt thereof of the present invention. Some compounds have a tendency to trap a fixed molar ratio of solvent molecules in the crystalline solid state, thus forming a solvate. The material formed by crystallization of the compound or a salt thereof of the present invention and the solvent in a three-dimensional order is called a solvate herein. The solvent can be associated with a crystalline solid form of the compound or a salt thereof of the present invention in various ways. The interaction can be due to weak binding (e.g., hydrogen bonding, van der Waals force, and dipole-dipole interaction) or by entrapment (e.g., liquid inclusion).
A solvate can be formed by a variety of methods, many of which are known in the art. The compound or a salt thereof of the present invention can be combined with one or more solvents by any suitable method (e.g., crystallization, lyophilization, film coating, spray drying, suspension, wetting, grinding, vapor sorption, etc.). For example, the compound or a salt thereof of the present invention can be combined with a particular solvent(s) and heated to boiling. The solution can then be slowly cooled to allow formation of the solvate crystals. Cooling can occur at room temperature or at a lower temperature (e.g., an ice bath and/or refrigerated conditions). Controlling the temperature can be influential in the formation of solvates. Typically, a lower temperature favors solvate formation. The formed solvate can be characterized by analytical methods such as thermogravimetric analysis (TGA), differential scanning calorimetry (DSC) alone or with infrared spectrophotometry (IR) and/or mass spectrometry, x-ray powder diffraction, moisture sorption experiments, hot-stage polarized light microscopy, or a combination of these methods. Various techniques to prepare solvates are known in the art. See, e.g., J. Keith Guillory, "Generation of Polymorphs, Hydrates, and Solvates, and Amorphous Solids," Drugs and the Pharmaceutical Sciences, 95 (Polymorphism in Pharmaceutical Solids): 183-226 (1999); and Greisser, U., "The Importance of Solvates" in Polymorphism, Hilfiker, R., Ed., (Wiley-VCH Verlag GmbH & Co. KGaA: Weinheim, Germany, 2006), pages 211-233.
A solvate means a solvent addition form that contains either stoichiometric or non-stoichiometric amounts of solvent. A stoichiometric solvate implies a fixed, although not necessarily integral, ratio of solvent to the compound or a salt thereof of the present invention (e.g., a solvent coordination number of 1, 2, 3, 4, 5, 6, etc.). A preferred solvent coordination number of a stoichiometric solvate is 1. A non-stoichiometric solvate can be an interstitial solid solution or an interstitial co-crystal. The solvent content of a solvate can be any suitable value, including a multiple of the molar compound ratio such that the solvent coordination number is a non-integral number (e.g., 0.1, 0.2, 0.3, 0.4, 0.5, 0.6, 0.7, 0.8, 0.9, 1.1, 1.2, 1.3, 1.4, 1.5, 1.6, 1.7, 1.8, 1.9, etc.). The amount of solvent in the structure generally depends on the partial pressure of the solvent in the environment of the solid and the temperature (Greisser, U., "The Importance of Solvates" in Polymorphism, Hilfiker, R., Ed., (Wiley-VCH Verlag GmbH & Co. KGaA: Weinheim, Germany, 2006), pages 211-233).
The solvent can be any suitable solvent, i.e., the solvent is not particularly limited as long as a solvate of the compound or a salt thereof of the present invention can be formed. Solvents usable for solvate formation include water, alcohols, ethers, esters, alkanes, benzene, dichloromethane, chloroform, acetone, acetonitrile, toluene, tetrahydrofuran, pyridine, dimethylformamide (DMF), dimethyl sulfoxide (DMSO), dioxane, and combinations thereof. In some embodiments, the solvate contains a mixture of solvents, such as a combination of two or more of the aforementioned solvents. Preferred solvents include water, alcohols, ethers, esters, and alkanes. If the solvent is water, the solvate formed is a "hydrate," whereas when the solvent is alcohol, the solvate formed is an "alcoholate." Specific examples of preferred solvents usable for solvate formation include water, C₁₋₄ alcohol (e.g., methanol, ethanol, propanol, isopropanol, and n-butanol), C₁₋₄ ether (e.g., diethyl ether), an ester of a C₁₋₆ (preferably C₁₋₄) alkyl acetate (e.g., methyl acetate, ethyl acetate, propyl acetate, and butyl acetate), a C₅₋₇ alkane (e.g., pentane, hexane, and heptane), and combinations thereof. Mixed solvents include, for example, water/ethanol, water/methanol, water/acetone, water/hexane, and water/DMF.

The production method of the present invention is explained below. It is needless to say that the present invention is not particularly limited to the production method below.
For the production of the present invention, the order of the reaction can be appropriately changed. The reaction only needs to be carried out from a step or position that seems to be reasonable to start from.
In addition, a step for appropriately transforming substituents (change or further modification of substituents) may be inserted between respective steps. When a reactive functional group is involved, appropriate protection or deprotection may be conducted. To promote progress of the reaction, moreover, reagents other than those exemplified can be appropriately used. The compound obtained in each step can be isolated and purified by conventional methods such as distillation, recrystallization, column chromatography and the like. In some cases, it is possible to proceed to the next step without isolation and purification.
In the production method, the "1 volume per 1 weight" means, for example, 1 L per 1 kg.

### Step 1

### Production method of Compound II

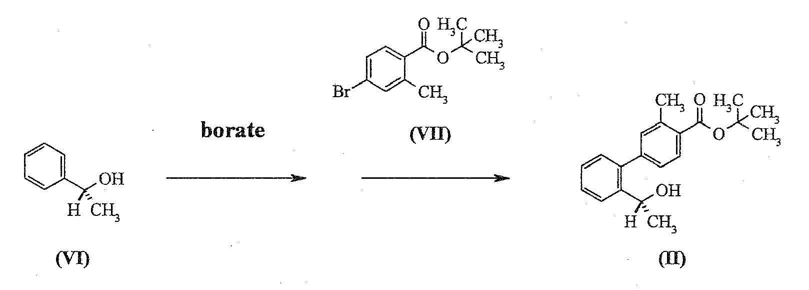

This step is a step comprising
(1-i) a step of preparing a dianion of (R)-1-phenylethanol (hereinafter sometimes to be abbreviated as Compound VI) from Compound VI, and subjecting the dianion to borylation with a borate, and
(1-ii) a step of reacting the borylated compound with tert-butyl 4-bromo-2-methylbenzoate (hereinafter sometimes to be abbreviated as Compound VII),
to prepare Compound II.

### Step (1-i)

A dianion of Compound VI is prepared from Compound VI, and the dianion is subjected to borylation with a borate.
The preparation of the dianion is preferably carried out in a solvent in the presence of a base, and in the presence of or in the absence of an additive.
Examples of the solvent used for the preparation of the dianion include ether solvents such as diethyl ether, tert-butyl methyl ether, di-n-butyl ether, cyclopentyl methyl ether and the like; hydrocarbon solvents such as n-hexane, toluene and the like, and the like. These solvents may be used in a mixture of two or more kinds thereof. Preferable solvent for this reaction is n-hexane. While the amount of the solvent to be used is not particularly limited, it is generally about 1 volume to about 100 volume, preferably about 5 volume to about 20 volume, per 1 weight of Compound VI.
Examples of the base used for the preparation of the dianion include alkyllithiums such as n-butyllithium, s-butyllithium and the like; alkali metal amides such as lithiumdiisopropylamide, sodium amide, lithiumbistrimethylsilylamide and the like, and the like. Of these, n-butyllithium is preferable. The amount of the base to be used is generally about 2 mol to about 5 mol, preferably about 2 mol to about 3 mol, per 1 mol of Compound VI.
Examples of the additive used for the preparation of the dianion include tertiary organic amines such as 1,4-diazabicyclo[2.2.2]octane (hereinafter sometimes to be abbreviated as DABCO), N,N,N',N'-tetramethylethylenediamine (hereinafter sometimes to be abbreviated as TMEDA), hexamethylphosphoric triamide and the like. Of these, TMEDA is preferable. The amount of the additive to be used is generally 0 mol to about 4 mol, preferably about 0.5 mol to about 2 mol, per 1 mol of Compound VI.
The reaction temperature for the preparation of the dianion is generally about -50°C to about 150°C, preferably room temperature to about 80°C.
The reaction time for the preparation of the dianion is generally about 30 min to about 4 days, preferably about 1 hr to about 24 hr.
The dianion thus prepared is generally subjected to the next borylation in the form of the reaction mixture after completion of the reaction, or after concentration. Preferably, it is subjected to the next borylation in the form of the reaction mixture after completion of the reaction.

Then, the above-mentioned dianion is subjected to borylation with a borate. This borylation is preferably carried out in a solvent.
Examples of the solvent used for the borylation include ether solvents such as diethyl ether, tert-butyl methyl ether, di-n-butyl ether, cyclopentyl methyl ether and the like; hydrocarbon solvents such as n-hexane, toluene and the like, and the like. These solvents may be used in a mixture of two or more kinds thereof. Preferable solvent for this reaction is n-hexane. While the amount of the solvent to be used is not particularly limited, it is generally about 1 volume to about 100 volume, preferably about 5 volume to about 20 volume, per 1 weight of Compound VI. When the dianion prepared in the above-mentioned step is used in the form of the reaction mixture after completion of the reaction, a solvent may be newly added. In this case, the solvent may be different from the solvent used for the preparation of the dianion. When the dianion prepared in the above-mentioned step is used after concentration, a solvent different from the solvent used for the preparation of the dianion may be added.
Examples of the borate used for the borylation include trimethyl borate, triethyl borate, triisopropyl borate, tri-n-butyl borate and the like. Of these, triisopropyl borate and tri-n-butyl borate are preferable. The amount of the borate to be used is generally about 0.5 mol to about 20 mol, preferably about 0.5 mol to about 5 mol, per 1 mol of Compound VI.
The reaction temperature for the borylation is generally about -100°C to about 150°C, preferably about -50°C to about 80°C .
The reaction time for the borylation is generally about 30 min to about 4 days, preferably about 1 hr to about 12 hr.
After completion of the reaction, the reaction mixture is treated according to a conventional method and, where necessary, purified, and the obtained compound is subjected to the next step.

### Step (1-ii)

The borylated compound obtained in Step (1-i) is reacted with Compound VII to prepare Compound II.
This reaction is preferably carried out in a solvent in the presence of a base and a metal catalyst according to the Suzuki coupling.
Examples of the solvent used for the reaction include ether solvents such as diethyl ether, tetrahydrofuran, 1,2-dimethoxyethane, diglyme, anisole and the like; hydrocarbon solvents such as hexane, benzene, toluene and the like; alcohol solvents such as methanol, ethanol, 1-propyl alcohol, tert-butanol and the like; ester solvents such as ethyl acetate, tert-butyl acetate and the like; polar solvents such as acetone, N,N-dimethylformamide, dimethyl sulfoxide, water and the like, and the like. These solvents may be used in a mixture of two or more kinds thereof. Preferable solvent for this reaction is a mixed solvent of water and tetrahydrofuran. While the amount of the solvent to be used is not particularly limited, it is generally about 1 volume to about 100 volume, preferably about 5 volume to about 20 volume, per 1 weight of Compound VII.
While the base used for the reaction is not particularly limited as long as it is a known base used for the Suzuki coupling, examples thereof include organic bases such as triethylamine, pyridine and the like; alkali metal hydroxides and alkaline earth metal hydroxides such as sodium hydroxide, potassium hydroxide and the like; alkali metal carbonates and alkaline earth metal carbonates such as sodium carbonate, potassium carbonate, sodium hydrogen carbonate, potassium hydrogen carbonate and the like; alkali metal phosphates and alkaline earth metal phosphates such as trisodium phosphate, tripotassium phosphate, disodium phosphate, dipotassium phosphate and the like; alkali metal carboxylates and alkaline earth metal carboxylates such as sodium acetate, potassium acetate and the like; alkali metal alkoxides and alkaline earth metal alkoxides such as sodium methoxide, potassium tert-butoxide and the like, and the like. Of these, tripotassium phosphate is preferable. The amount of the base to be used is generally about 0.3 mol to about 20 mol, preferably about 1 mol to about 5 mol, per 1 mol of Compound VII.
While the metal catalyst used for the reaction is not particularly limited as long as it is a known metal catalyst having a catalyst action shown in the Suzuki coupling, a palladium catalyst is generally used. Examples thereof include palladium chloride (PdCl₂), palladium acetate (Pd(OAc)₂), bis(triphenylphosphine)palladium dichloride (PdCl₂(PPh₃)₂), bis(dibenzylideneacetone)palladium (Pd(dba)₂), tris(dibenzylidene)dipalladium (Pd₂(dba)₃), tetrakis(triphenylphosphine)palladium (Pd(PPh₃)₄), palladium carbon (Pd-C) and the like. These catalysts may be used in a mixture of two or more kinds thereof. In addition, the catalyst prepared in the reaction system may be used without purification. Preferable metal catalyst for this reaction is bis(triphenylphosphine)palladium dichloride (PdCl₂(PPh₃)₂). The amount of the metal catalyst to be used is generally about 0.01 %mol to about 20 %mol, preferably about 0.05 %mol to about 1 %mol, per 1 mol of Compound VII.
In the reaction, the above-mentioned metal catalyst may be used together with a ligand, and examples of the ligand include triphenylphosphine, tri-tert-butylphosphine, di-tert-butylmethylphosphine, tricyclohexylphosphine and the like. While the amount of the ligand to be used is not particularly limited, it is preferably 0 to 5 ligands per the metal element of the catalyst.
The reaction temperature for the reaction is generally about -50°C to about 200°C, preferably about 50°C to about 100°C.
The reaction time for the reaction is generally about 1 hr to about 24 hr, preferably about 2 hr to about 10 hr.
After completion of the reaction, the reaction mixture is treated according to a conventional method and, where necessary, purified to give Compound II. In addition, where necessary, the compound may be crystallized using seed crystals of Compound II.

### Step 2

### Production method of Compound III

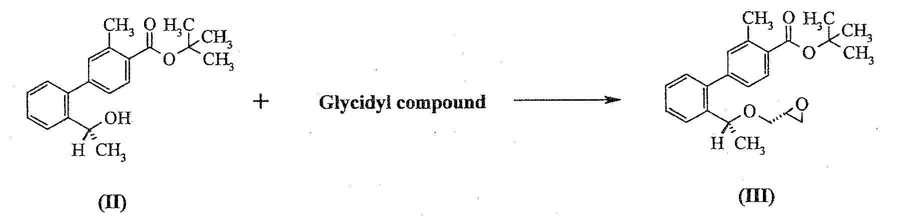

This step is a step of subjecting Compound II to glycidylation to prepare Compound III.

The glycidylation is preferably carried out using a glycidyl compound or a solvate thereof in a solvent in the presence of a base.
The glycidyl compound means a compound having a glycidyl group, and examples thereof include (R)-glycidyl 3-nitrobenzenesulfonate (alias (R)-glycidyl nosylate), (R)-glycidyl 4-methylbenzenesulfonate (alias (R)-glycidyl tosylate), (S)-epichlorohydrin and the like. Of these, (R)-glycidyl 3-nitrobenzenesulfonate and (S)-epichlorohydrin are preferable. The amount of the glycidyl compound or a solvate thereof to be used is generally about 1 mol to about 5 mol, preferably about 1 mol to about 2 mol, per 1 mol of Compound II.
Examples of the solvent used for the reaction include ether solvents such as diethyl ether, tetrahydrofuran, dioxane, 1,2-dimethoxyethane, diglyme, anisole and the like; hydrocarbon solvents such as benzene, toluene, hexane, xylene and the like; ester solvents such as ethyl acetate, tert-butyl acetate and the like; polar solvents such as acetone, N,N-dimethylformamide, dimethyl sulfoxide, water and the like, and the like. These solvents may be used in a mixture of two or more kinds thereof. When (S)-epichlorohydrin is used as a glycidyl compound or a solvate thereof, the solvent is preferably a mixed solvent of water and the above-mentioned organic solvent, more preferably a mixed solvent of water and toluene. When (R)-glycidyl 3-nitrobenzenesulfonate is used as a glycidyl compound, the solvent is preferably the above-mentioned organic solvent, more preferably 1,2-dimethoxyethane or diglyme. While the amount of the solvent to be used is not particularly limited, it is generally about 1 volume to about 50 volume, preferably about 2 volume to about 10 volume, per 1 weight of Compound II.
Examples of the base used for the reaction include organic bases such as triethylamine, pyridine and the like; alkali metal hydroxides and alkaline earth metal hydroxides such as sodium hydroxide, potassium hydroxide and the like; alkali metal carbonates and alkaline earth metal carbonates such as sodium carbonate, potassium carbonate, sodium hydrogen carbonate, potassium hydrogen carbonate and the like; alkali metal hydrides and alkaline earth metal hydrides such as sodium hydride, potassium hydride and the like; alkyllithiums such as n-butyllithium, s-butyllithium and the like, and the like. Of these, sodium hydride and sodium hydroxide are preferable. The amount of the base to be used is generally about 1 mol to about 20 mol, preferably about 1 mol to about 5 mol, per 1 mol of Compound II.
The reaction temperature for the reaction is generally about 0°C to about 150°C, preferably about 15°C to about 100°C.
The reaction time for the reaction is generally about 1 hr to about 4 days, preferably about 5 hr to about 2 days.
After completion of the reaction, the reaction mixture is treated according to a conventional method and, where necessary, purified to give Compound III.

### Step 3

### Production method of Compound V

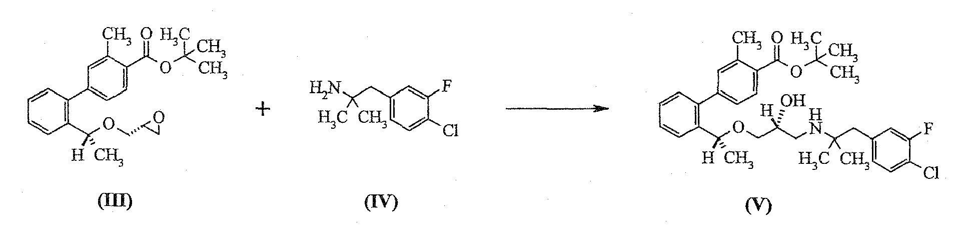

This step is a step of reacting Compound III with Compound IV to prepare Compound V.

This reaction is preferably carried out in a solvent or without a solvent.
When a solvent is used for the reaction, examples thereof include ether solvents such as diethyl ether, tetrahydrofuran, 1,2-dimethoxyethane, cyclopentyl methyl ether and the like; hydrocarbon solvents such as hexane, benzene, toluene and the like; halogenated hydrocarbon solvents such as methylene chloride, chloroform and the like; alcohol solvents such as methanol, ethanol, 1-propyl alcohol, tert-butanol and the like; ester solvents such as ethyl acetate, tert-butyl acetate and the like; polar solvents such as acetone, N,N-dimethylformamide, dimethyl sulfoxide, water and the like, and the like. These solvents may be used in a mixture of two or more kinds thereof. Preferable solvent for this reaction is methanol or toluene. While the amount of the solvent to be used is not particularly limited, it is generally 0 volume to about 100 volume, preferably about 2 volume to about 10 volume, per 1 weight of Compound III.
The amount of Compound IV to be used is generally about 1 mol to about 5 mol, preferably about 1 mol to about 2 mol, per 1 mol of Compound III.
The reaction temperature for the reaction is generally about 15°C to about 200°C, preferably about 50°C to about 150°C.
The reaction time for the reaction is generally about 2 hr to about 4 days, preferably about 5 hr to about 24 hr.
After completion of the reaction, the reaction mixture is treated according to a conventional method and, where necessary, purified to give Compound V. Alternatively, the reaction mixture may be subjected to the next step without purification.

### Step 4

### Production method of Compound I

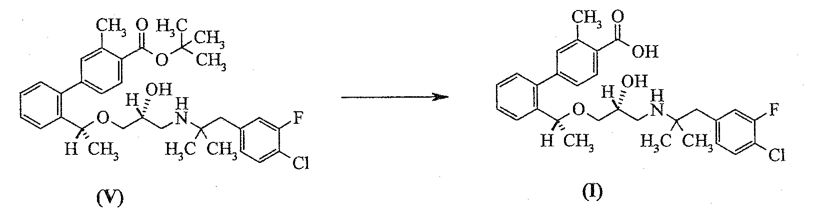

This step is a step of (i) subjecting Compound V to alkali hydrolysis, or (ii) subjecting Compound V to transesterification and then to alkali hydrolysis, to Compound I.

### (i) Method by alkali hydrolysis

The alkali hydrolysis is preferably carried out in water or a mixed solvent of water and an organic solvent, in the presence of a base.
While the solvent used for the alkali hydrolysis is not particularly limited as long as it is a solvent generally used for alkali hydrolysis, examples thereof include water and a mixed solvent of water and an organic solvent. Examples of the organic solvent include ether solvents such as diethyl ether, tetrahydrofuran, 1,2-dimethoxyethane, cyclopentyl methyl ether and the like; hydrocarbon solvents such as hexane, benzene, toluene and the like; alcohol solvents such as methanol, ethanol, 1-propyl alcohol and the like; polar solvents such as N,N-dimethylformamide, dimethylsulfoxide and the like, and the like. These solvents may be used in a mixture of two or more kinds thereof and water. Preferable solvent for this reaction is an alcohol solvent, particularly methanol. The amount of the organic solvent to be used is generally about 1 volume to about 100 volume, preferably about 2 volume to about 10 volume, per 1 weight of Compound V. The amount of the water to be used is about 0.05 volume to about 2 volume per 1 volume of the organic solvent to be used.
The base used for the alkali hydrolysis includes alkali metal hydroxides such as lithium hydroxide, sodium hydroxide, potassium hydroxide and the like. Of these, sodium hydroxide and potassium hydroxide are preferable. The amount of the base to be used is generally about 1 mol to about 20 mol, preferably about 1 mol to about 5 mol, per 1 mol of Compound V.
The reaction temperature for the alkali hydrolysis include is generally room temperature to about 200°C, preferably about 50°C to about 100°C.
The reaction time for the alkali hydrolysis include is generally about 3 hr to about 7 days, preferably about 10 hr to about 3 days.

### (ii) Method by transesterification and then alkali hydrolysis.

The transesterification is preferably carried out with an alkali metal alkoxide in an organic solvent.
Examples of the alkali metal alkoxide used for the transesterification include alkali metal alkoxides derived from primary alcohol or secondary alcohol, such as sodium methoxide, potassium isopropoxide and the like, preferably alkali metal alkoxides derived from primary alcohol, particularly preferably sodium methoxide. The amount of the alkali metal alkoxide to be used is generally about 0.1 mol to about 20 mol, preferably about 1 mol to about 5 mol, per 1 mol of Compound V. The alkali metal alkoxide prepared in the reaction system may be used.
Examples of the organic solvent used for the transesterification include ether solvents such as diethyl ether, tetrahydrofuran, 1,2-dimethoxyethane, cyclopentyl methyl ether and the like; hydrocarbon solvents such as hexane, benzene, toluene and the like; alcohol solvents such as methanol, ethanol, 1-propyl alcohol and the like; polar solvents such as N,N-dimethylformamide, dimethylsulfoxide and the like, and the like. These solvents may be used in a mixture of two or more kinds thereof. Preferable examples of the combination of the solvent and the alkali metal alkoxide for this reaction include a combination of methanol and sodium methoxide, and a combination of ethanol and sodium ethoxide. Preferable solvent for this reaction is methanol. The amount of the organic solvent to be used is generally about 1 volume to about 100 volume, preferably about 2 volume to about 10 volume, per 1 weight of Compound V.
The reaction temperature for the transesterification is generally room temperature to about 200°C, preferably about 50°C to about 100°C.
The reaction time for the transesterification is generally about 2 hr to about 4 days, preferably about 5 hr to about 2 days.

After the transesterification, the resulting compound is subjected to alkali hydrolysis. The alkali hydrolysis is carried out by adding water to the reaction solution after the transesterification. The amount of the water to be used is generally about 0.05 volume to about 2 volume per 1 volume of the organic solvent used for the transesterification.
The reaction temperature for the alkali hydrolysis is generally room temperature to about 200°C, preferably about 50°C to about 100°C.
The reaction time for the alkali hydrolysis is generally about 10 min to about 4 days, preferably about 1 hr to about 24 hr.
Preferable embodiment of this step is (ii) method by transesterification and then alkali hydrolysis.
After completion of the reaction, the reaction mixture is treated according to a conventional method and, where necessary, purified to give Compound I. In addition, where necessary, the compound may be crystallized using seed crystals of Compound I.

### Examples

In the following examples, abbreviations may sometimes be used:
TMEDA: N,N,N',N'-tetramethylethylenediamine
Si-Thiol: mercaptopropyl functionalized silica gel
DME: 1,2-dimethoxyethane
THF: tetrahydrofuran

### Example 1

### Production of Compound II

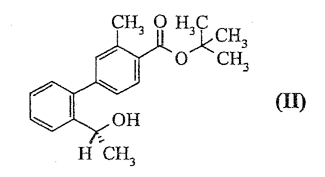

### Step 1

Production of mixture of tert-butyl 4-dihydroxyboryl-2-methylbenzoate (hereinafter sometimes to be abbreviated as Compound VIII) and tris(4-tert-butyloxycarbonyl-3-methylphenyl)boroxin (hereinafter sometimes to be abbreviated as Compound IX).

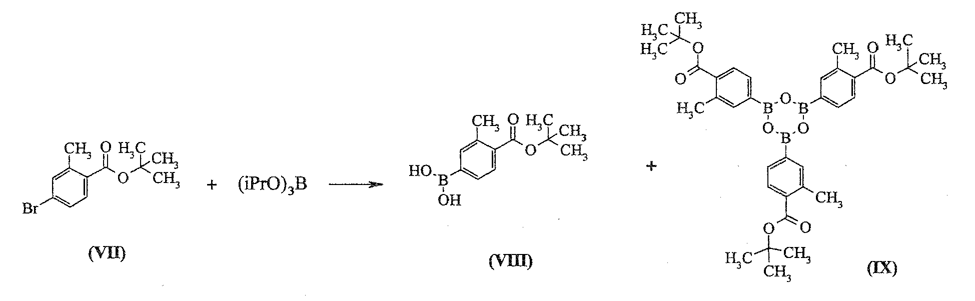

To a solution of triisopropyl borate (73.7 g) and Compound VII (96.5 g) in THF (0.48 L) was added dropwise 1.6 mol/L n-butyllithium/n-hexane solution (0.25 L) at -60°C to - 70°C under a nitrogen atmosphere, and the mixture was stirred at the same temperature for 1 hr, and allowed to warm to -10°C. 2N Hydrochloric acid (0.23 L) was added to the reaction mixture, and the organic layer was washed with water (0.24 L, twice), and concentrated under reduced pressure. Toluene (0.29 L) was added to the residue, and the mixture was concentrated under reduced pressure. Toluene (0.19 L) was again added to the residue, the mixture was warmed to 75°C, and n-heptane (0.19 L) was added dropwise. The mixture was allowed to cool to room temperature, and the precipitate was collected by filtration, and dried under reduced pressure to give a mixture (44.9 g, yield 53.4%) containing Compound VIII and Compound IX. ¹H-NMR ( 400Mz, DMSO-d₆, δ) :1.54 (s,2H), 1.55(s,7H), 2.47(s,0.7H), 2.53(s,2.3H), 7.66-7.77(m,3H), 8.19(broad s,0.4H)

### Step 2

### Production of tert-butyl 3-methyl-2'-[(2R)-1-acetyloxyethyl]biphenyl-4-carboxylate (hereinafter sometimes to be abbreviated as Compound XII)

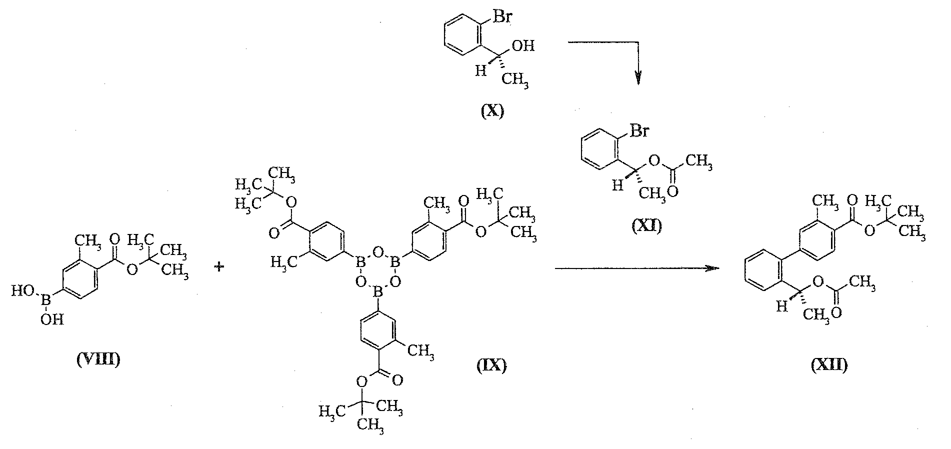

To a solution of (R)-1-(2-bromophenyl)ethanol (hereinafter sometimes to be abbreviated as Compound X) (20.0 g) in toluene (100 mL) were successively added triethylamine (16.5 mL), acetic anhydride (10.3 mL) and 4-dimethylaminopyridine (608 mg) under a nitrogen atmosphere, and the mixture was stirred at room temperature for 1 hr. Ice water (60 mL) was added, and the organic layer was washed with water (60 mL, twice), and concentrated under reduced pressure. Tetrahydrofuran (100 mL) was added to the residue, and the mixture was concentrated under reduced pressure. These operations were performed twice. THF (120 mL), water (40 mL) and the mixture (24.6 g) containing Compound VIII and Compound IX were added to the residue under a nitrogen atmosphere, and the mixture was vigorously stirred at room temperature for 10 min. Then bis(triphenylphosphine)dichloropalladium (698 mg) was added, and then a solution of tripotassium phosphate (31.6 g) in water (80 mL) was added dropwise at 60 to 70°C. The mixture was stirred at the same temperature for 4 hr, and allowed to cool to room temperature, and the organic layer was separated. Toluene (100 mL) was added to the organic layer, and the organic layer was washed successively with a mixture (twice) of diethylenetriamine (10.3 g) and water (120 mL), a mixture (once) of acetic acid (6 mL) and water (120 mL), and water (120 mL, once), and concentrated under reduced pressure. Methanol (120 mL) and Si-Thiol (2 g) were added to the residue, and the mixture was stirred at room temperature for 1 hr. The solution was filtered, and the filtrate was concentrated under reduced pressure. The residue was purified by silica gel column chromatography (n-hexane:ethyl acetate=1:4) to give the title compound. The total amount thereof was used for the next step.

### Step 3

### Production of Compound II

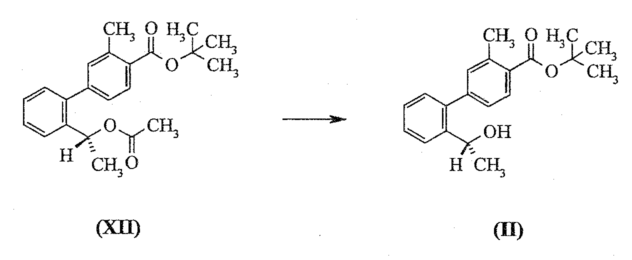

Methanol (80 mL) was added to Compound XII obtained in the above-mentioned step under a nitrogen atmosphere, and 4N aqueous sodium hydroxide solution (27.3 mL) and THF (80 mL) were successively added under ice-cooling. The mixture was stirred at the same temperature for 1.5 hr, acetic acid (1.14 mL) was added to the reaction mixture, and the reaction mixture was concentrated under reduced pressure to a half volume. The solution was extracted with n-hexane (150 mL) and ethyl acetate (150 mL), and the organic layer was dried over anhydrous magnesium sulfate, and concentrated under reduced pressure. n-Heptane (400 mL) was added to the residue, and the precipitated solid was collected by filtration. The same operations were performed twice for mother liquor. These solids were combined, and dried under reduced pressure to give the title compound (crystals, 27.2 g, yield from Compound X: 87.5%) .
¹H-NMR (400Mz, DMSQ-d₆, δ) :1.19(d,J=6.4Hz,3H), 1.57(s,9H), 2.54(s,3H), 4.69-4.75(m,1H), 5.07(d,J=4.0Hz,1H), 7.11-7.13(m,1H), 7.22-7.24(m,2H), 7.28-7.32(m,1H), 7.39-7.43(m,1H), 7.64-7.66(m,1H), 7.78-7.80(m,1H)

### Example 2

### Production of 2'-((1R)-1-{(2R)-3-[1-(4-chloro-3-fluorophenyl)-2-methylpropan-2-ylamino]-2-hydroxypropoxy}ethyl)-3-methylbiphenyl-4-carboxylic acid 1/2 sulfate 1/2 hydrate (hereinafter sometimes to be abbreviated as Compound I-a)

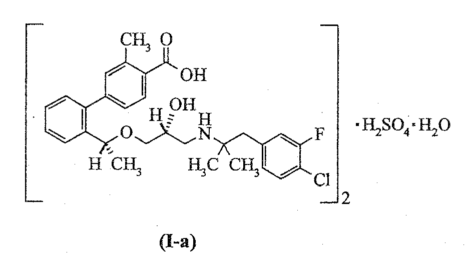

### Step 1

### Production of Compound II

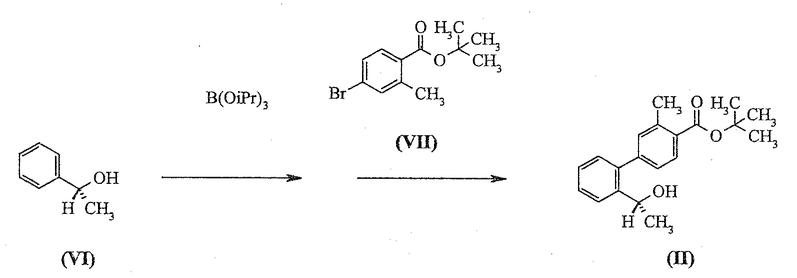

To a 1.6 mol/L n-butyllithium/n-hexane solution (7.22 L) were successively added dropwise Compound VI (632 g), n-hexane (0.63 L) and TMEDA (720 g) under ice-cooling under a nitrogen atmosphere. The mixture was stirred at 50°C for 1 hr, and allowed to cool to room temperature. The solution was added dropwise to triisopropyl borate (1.94 kg) at room temperature under a nitrogen atmosphere. The mixture was stirred at 40°C for 2 hr. A mixture of 35 w/w% hydrochloric acid (2.16 kg) and water (2.5 L) was added dropwise to the reaction mixture under ice-cooling, and the organic layer was separated at room temperature. The organic layer was washed with 10 w/w% brine (1.9 L), and concentrated under reduced pressure to give a residue (959 g).
To the obtained residue (959 g) were added THF (4.8 L), water (1.8 L) and Compound VII (1.00 kg) under a nitrogen atmosphere, and the mixture was vigorously stirred at room temperature for 10 min. Bis(triphenylphosphine)palladium dichloride (13.0 g) was added, and then a solution of tripotassium phosphate (1.18 kg) in water (3.0 L) was added dropwise over 1 hr at 65°C. The mixture was stirred at the same temperature for 1.5 hr, and allowed to cool to room temperature, and the organic layer was separated. Toluene (5.0 L) was added to the organic layer, and the organic layer was washed successively with a mixture (twice) of diethylenetriamine (381 g) and water (5.0 L), a mixture of acetic acid (0.25 L) and water (5.0 L), and water (5.0 L), and concentrated under reduced pressure. Methanol (5.0 L) was added to the residue, and the mixture was concentrated under reduced pressure. These operations were performed twice. Methanol was added to the residue to a total volume of 5.0 L, Si-Thiol (15.3 g) was added, and the mixture was stirred overnight at room temperature. The solution was filtered, and the filtered substance was washed with methanol (3.4 L). The filtrate and the washing solution were combined, and water (1.8 L) was added dropwise thereto at room temperature. Seed crystals (0.15 g) were added, and the mixture was stirred at the same temperature for 70 min. Water (3.0 L) was added dropwise again at room temperature, and the mixture was stirred overnight at the same temperature. The precipitated crystals were collected by filtration, washed with a mixture of methanol (2.2 L) and water (1.4 L), and dried under reduced pressure to give the title compound (crystals, 1.10 kg, yield from Compound VII: 95.5%).
¹H-NMR (400Mz, DMSO-d₆, δ) :1.18(d,J=6.3Hz,3H), 1.55(s,9H), 2.52(s,3H), 4.68-4.73(m,1H), 5.05(d,J=4.2Hz,1H), 7.10-7.12(m,1H), 7.21-7.23(m,2H), 7.26-7.30(m,1H), 7.38-7.42(m,1H), 7.62-7.65(m,1H), 7.77-7.79(m,1H)
The crystals of Compound II used as seed crystals may be, for example, crystals prepared by the method of Example 1, or crystals prepared in advance by Step 1.
Since Compound II can be obtained as a crystal, a production method improved in the easiness of quality control can be provided.

### Step 2

### Production of Compound III

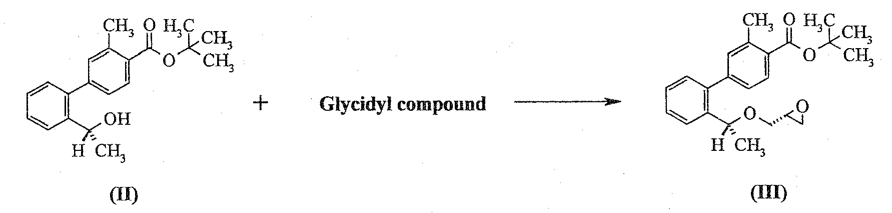

To a solution of 60% sodium hydride (109 g) in DME (0.75 L) was added dropwise a solution of Compound II (500 g) and (R)-glycidyl 3-nitrobenzenesulfonate (498 g) in DME (1.0 L) under ice-cooling under a nitrogen atmosphere. The dropping funnel was washed with DME (0.25 L), and the washing solution was also added dropwise under ice-cooling. After the completion of the dropwise addition, the mixture was stirred overnight at room temperature. Water (2.0 L) was added dropwise to the reaction mixture under ice-cooling, the mixture was extracted with toluene (2.0 L) at room temperature, and the organic layer was separated. The organic layer was washed with 10 w/w% brine (2.0 L, twice), and concentrated under reduced pressure. Methanol (2.0 L) was added to the residue, and the mixture was concentrated under reduced pressure. These operations were performed twice. The methanol was added to the residue to a total volume of 2.0 L, and the solution was used for the next step.
The crude product obtained in the same manner as in this step was purified by silica gel column chromatography (n-hexane:ethyl acetate=5:1) to give the title compound.
1H-NMR (400Mz, DMSO-d₆, δ) :1.27(d,J=6.4Hz,3H), 1.57(s,9H), 2.43(dd,J=2.7,5.1Hz,1H), 2.54(s,3H), 2.65(dd,J=4.2,5.1Hz,1H), 2.97-3.05(m,2H), 3.43(dd,J=2.4,11.0Hz,1H), 4.49(q,J=6.4Hz,1H), 7.17(dd,J=1.1,7.5Hz,1H), 7.20-7.22(m,2H), 7.33-7.37(m,1H), 7.44-7.48(m,1H), 7.54-7.57(m,1H), 7.80(d,J=7.7Hz,1H)
Using Compound II, a production method improved from the aspects of progress of glycidylation reaction can be provided.

### Step 3

### Production of Compound V

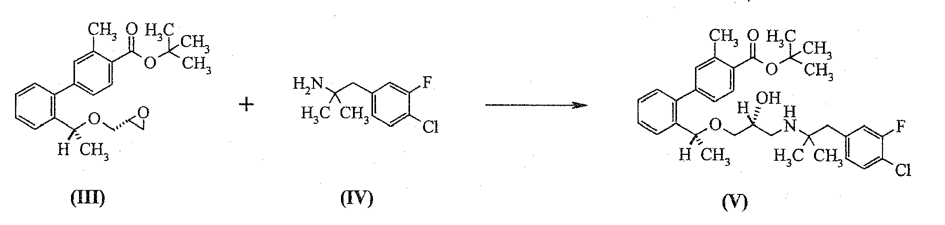

To the methanol solution (1.0 L) obtained in the above-mentioned step was added Compound IV (194 g) under a nitrogen atmosphere, and the mixture was stirred overnight with refluxing. The same operation was performed on the same scale, and two reaction mixtures were combined, and used for the next step.
The crude product obtained in the same manner as in this step was purified by silica gel column chromatography (n-hexane:ethyl acetate=2:1) to give the title compound.
¹H-NMR(400Mz,DMSO-d₆, δ) :0.89(s,3H), 0.91(s,3H), 1.26(d,J=6.3Hz,3H), 1.56(s,9H), 2.37-2.41(m,1H), 2.53-2.58(m,6H), 3.05-3.12(m,2H), 3.47-3.54(m,1H), 4.40-4.45(m,1H), 4.61(d,J=4.9Hz,1H), 7.00(dd,J=1.6,8.1Hz,1H), 7.15-7.21(m,4H), 7.32-7.46(m,3H), 7.52-7.54(m,1H), 7.79-7.81(m,1H)

### Step 4

### Production of Compound I

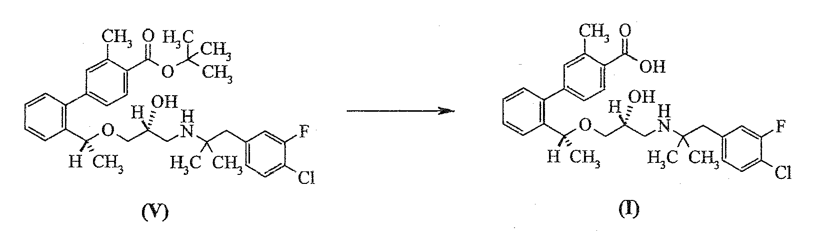

To the methanol solution obtained in the above-mentioned step was added a methanol solution (618 g, 28 w/w%) of sodium methoxide under a nitrogen atmosphere, and the mixture was stirred for 6 hr with refluxing. Water (0.50 L) was added, and the mixture was again stirred for 2 hr with refluxing. The mixture was allowed to cool to room temperature, and washed with n-heptane (1.0 L, twice), and the organic layer was separated. Methanol (80 mL) and water (0.30 L) were successively added to the lower layer (aqueous methanol solution), and acetic acid (202 g) was added dropwise at 45°C while stirring. Seed crystals (0.25 g) were added, and the mixture was stirred at the same temperature for 5 hr, and then at room temperature for 4 days. The precipitated crystals were collected by filtration, washed with a mixture of methanol (1.3 L) and water (0.40 L), and dried under reduced pressure to give the title compound (crystals, 579 g, yield from Compound II: 70.4%).
¹H-NMR(400Mz,DMSO-d₆,δ):1.01(s,3H), 1.03(s,3H), 1.27(d,J=6.3Hz,3H), 2.55-2.57(m,4H), 2.74-2.78(m,3H), 3.13(d,J=5.7Hz,2H), 3.67-3.70(m,1H), 4.46-4.51(m,1H), 7.03-7.05(m,1H), 7.13-7.18(m,3H), 7.22-7.26(m,1H), 7.32-7.36(m,1H), 7.41-7.46(m,2H), 7.54(dd,J=1.1,7.9Hz,1H), 7.80-7.82(m,1H) MS (ESI,m/z) 514(M+H)⁺
The crystals of Compound I used as seed crystals may be, for example, crystals prepared by the following method, or crystals prepared in advance by Step 4.
To Compound I (500 mg) described in WO2004/094362 was added methanol (5 ml), and the mixture was heated under reflux for 2 hr, and allowed to cool to room temperature. The precipitated crystals were collected by the filtration to the title compound (400 mg).

### Step 5

### Production of Compound I-a

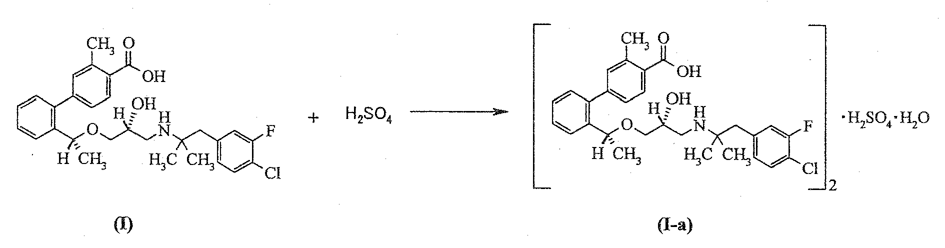

To Compound I (20.0 g) were successively added water (30 mL) and 1-propanol (0.12 L) under a nitrogen atmosphere, and the obtained suspension was warmed to 60°C. After the confirmation of dissolution, the solution was filtered, and the filtered substance was washed with a mixture of 1-propanol (24 mL) and water (6 mL). The filtrate and the washing solution were combined, and a mixture of 96 w/w% sulfuric acid (2.08 g) and water (20 mL), and water (0.20 L) were successively added dropwise thereto at 70°C under a nitrogen atmosphere, and then seed crystals (10 mg) were added thereto at the same temperature. The mixture was stirred at 60 to 70°C for 2.5 hr, allowed to cool to room temperature, and stirred overnight. The precipitated crystals were collected by filtration, washed successively with a mixture of 1-propanol (20 mL) and water (40 mL), and water (40 mL), and dried under reduced pressure to give the title compound (crystals, 19.4 g, yield 87.2%).
¹H-NMR(400Mz,DMSO-d₆,δ):1.09(s,3H),1.09(s,3H), 1.30(d,J=6.0Hz,3H), 2.57(s,3H), 2.62-2.67(m,1H), 2.83-2.90(m,3H), 3.14(d,J=5.3Hz,2H), 3.71-3.77(m,1H), 4.44-4.49(m,1H), 7.07(d,J=8.1Hz,1H), 7.18-7.20(m,3H), 7.27(d,J=10.5Hz,1H), 7.36(dd,J=7.4,7.4Hz,1H), 7.44-7.51(m,2H), 7.55(d,J=7.9Hz,1H), 7.88(d,J=7.9Hz,1H)
MS(ESI,m/z) 514(M+H)⁺
The crystals of Compound I-a used as seed crystals may be, for example, crystals prepared according to the method described in WO2004/094362, or crystals prepared in advance by Step 5.

## Claims

1. A compound having the structural formula: 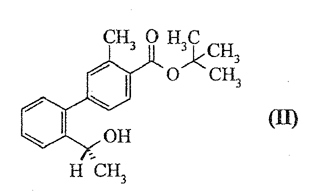 or a salt thereof.

2. A compound having the structural formula:

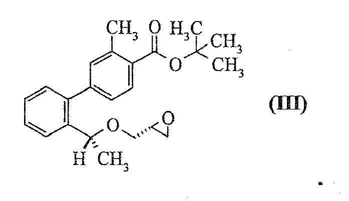

3. A compound having the structural formula: 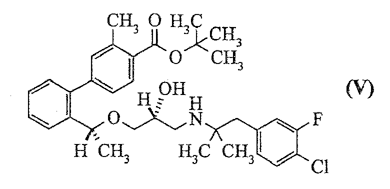 or a salt thereof.

4. A method for the preparation of a compound having the structural formula: 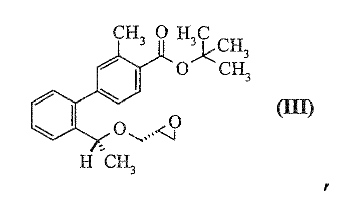 which comprises a step of subjecting a compound having the structural formula: 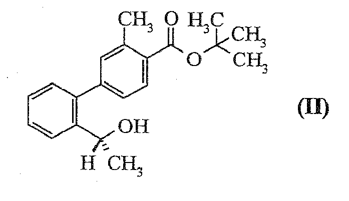 or a salt thereof to glycidylation.

5. A method for the preparation of a compound having the structural formula: 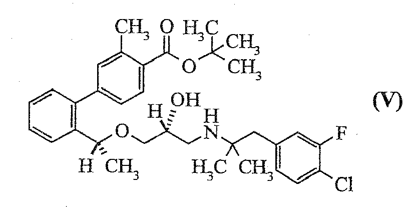 or a salt thereof, which comprises
(i) a step of subjecting a compound having the structural formula: 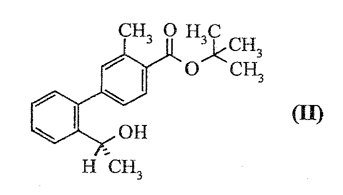 or a salt thereof to glycidylation to prepare a compound having the structural formula: 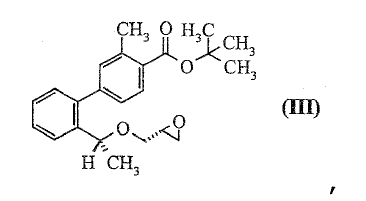 and
(ii) a step of reacting the formula III compound with a compound having the structural formula: 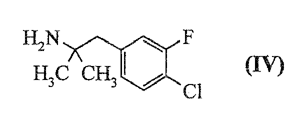
or a salt thereof.

6. A method for the preparation of a compound having the structural formula: 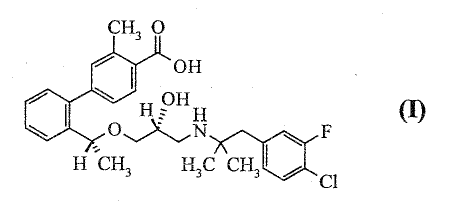 or a salt thereof, which comprises
(i) a step of subjecting a compound having the structural formula: 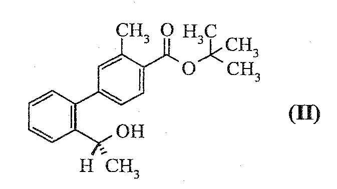 or a salt thereof to glycidylation to prepare a compound having the structural formula: 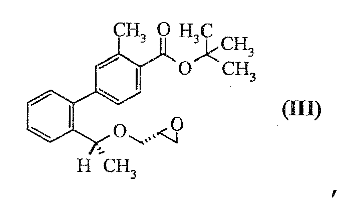
(ii) a step of reacting the formula III compound with a compound having the structural formula: 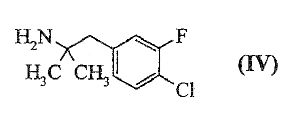 or a salt thereof to prepare a compound having the structural formula: 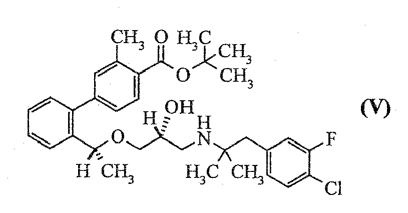 or a salt thereof, and
(iii) a step of subjecting the formula V compound or a salt thereof to alkali hydrolysis.

7. A method for the preparation of a compound having the structural formula: 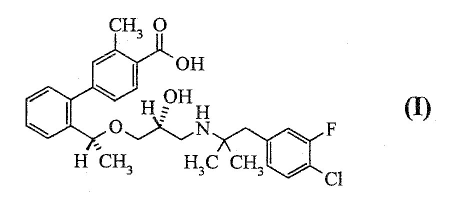 or a salt thereof, which comprises a step of subjecting a compound having the structural formula: 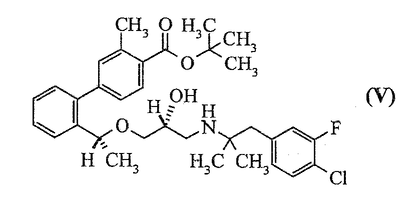 or a salt thereof to alkali hydrolysis.

8. The method of claim 6 or 7, wherein the step of subjecting a compound having the structural formula: 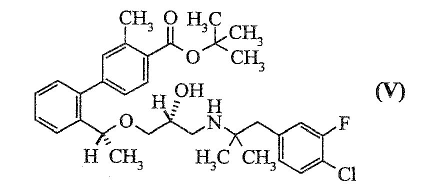 or a salt thereof to alkali hydrolysis is a step of subjecting a compound having the structural formula: 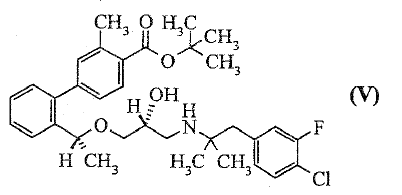 or a salt thereof to transesterification and then to alkali hydrolysis.
